# EUROPEAN PATENT SPECIFICATION

(11) **EP 3 558 259 B1**
(45) Date of publication and mention of the grant of the patent: **06.11.2024**
(21) Application number: 17823100.7
(22) Date of filing: 19.12.2017
(51) Int. Cl.: A61K 8/73, A61Q 19/00, A61K 8/81, A61K 8/96

(54) **COMPOSITION WITH A HIGH CONTENT OF SPRING AND/OR MINERAL WATER**
ZUSAMMENSETZUNG MIT EINEM HOHEN GEHALT AN QUELL- UND/ODER MINERALWASSER
COMPOSITION À HAUTE TENEUR EN EAU DE SOURCE ET/OU MINÉRALE

(30) Priority: 20.12.2016 FR 1662857
(43) Date of publication of application: 30.10.2019
(73) Proprietor: L'OREAL, 75008 Paris (FR)
(72) Inventor: LEGRET, Sylvie, 94152 Chevilly La Rue (FR); DE MARCO, Marine, 94152 Chevilly La Rue (FR)
(74) Representative: Cabinet Nony
(86) International application number: PCT/EP2017/083635
(87) International publication number: WO 2018/115005

(56) References cited:
- EP-A1- 2 208 510
- FR-A1- 3 000 670
- GB-A- 2 474 041
- ANONYMOUS: "GNPD - Advanced Filler", 1 May 2014 (2014-05-01), XP055349380, Retrieved from the Internet <URL:http://www.gnpd.com/sinatra/recordpage/2334867/from_search/9v3G9uLv5W/?page=2> [retrieved on 20170224]
- ANONYMOUS: "GNPD - Anti-Age Gel Cream SPF 20", 1 October 2016 (2016-10-01), XP055349384, Retrieved from the Internet <URL:http://www.gnpd.com/sinatra/recordpage/4375695/from_search/9v3G9uLv5W/?page=1> [retrieved on 20170224]
- COLLOBER I ET AL: "ACTIVITY OF VITTEL WATER ON PROLIFERATION OF HUMAN FIBROBLASTS PROLIFERATION AND DIFFERENTIATION OF HUMAN KERATINOCYTES", INTERNATIONAL JOURNAL OF COSMETIC SCIENCE, KLUWER ACADEMIC PUBLISHERS, DORDRECHT, NL, vol. 16, no. 4, 1 January 1994 (1994-01-01), pages 149 - 160, XP000953265, ISSN: 0142-5463

## Description

The present invention relates to the field of caring for keratin materials, more particularly that of facial care. It more particularly relates to compositions comprising hyaluronic acid, in the presence of a high content of spring and/or mineral water.

Hyaluronic acid is known for its film-forming and water-retaining power. Hyaluronic acid is a predominant glycosaminoglycan located in the skin. This compound, and the salts thereof, are conventionally used in cosmetics for their hygroscopic, viscoelastic and rheological properties, advantageously making it possible to moisturize and plump up the skin, and to reduce wrinkles and dehydration lines, with anti-aging effects. The hyaluronic acids most commonly used for the abovementioned applications are macromolecules of a molecular weight of between 500 kDa and 2 000 kDa. Indeed, it has been demonstrated that supplying these high-molecular-weight hyaluronic acids provides an effective moisturizing effect to the skin, by virtue of their hygroscopic properties which are characteristic of these molecules.

Spring and/or mineral waters, and in particular spring and/or mineral waters having a mineralization of at least 400 mg/l, are known for their soothing effect on the skin. They are commonly used in dermatological or cosmetic compositions at contents not exceeding 15% by weight relative to the total weight of the composition, due to the potential risk of depolymerization and crystallization of the minerals which they contain.

GB2474041A relates to cosmetic preparations comprising at least 75% of thermal mineral waters within the aqueous portion of the formulation. However, none of these cosmetic preparations are in the form of a single-phase composition and even less under the form of a gel. It has thus been observed that, in the presence of a high content of spring and/or mineral water, and due to its high mineralization, hyaluronic acid tends to cause phase separation of the composition containing it, which is of course a phenomenon to be avoided.

There therefore remains a need to provide care compositions, more particularly in the form of aqueous gels, or even of transparent gels, comprising hyaluronic acid and a high content of spring and/or mineral water.

Thus, according to a first of its aspects, the present invention relates to a composition according to claim 1.

Herein is described and not encompassed by the wording of the claims, a composition especially an aqueous composition, comprising hyaluronic acid or a salt thereof, spring and/or mineral water in a content greater than 20% by weight relative to the total weight of the composition, and at least one acrylic acid polymer, a copolymer of acrylic acid and of alkylacrylate, or one of the mixtures thereof.

The composition according to the present invention is in the form of a gel and in particular in the form of a transparent gel. In terms of sensory aspect, said gel may advantageously be qualified as lightweight gel texture, which is very well appreciated by the users. It may also be described as providing skin feeling fresh and not sticky or greasy-looking.

More precisely, the inventors have observed that the specific choice of one of these specific gelling polymers made it possible to mitigate the phase separation caused by the combination, within an aqueous composition, of hyaluronic acid or a salt thereof and of spring and/or mineral water in a content greater than 20% by weight relative to the total weight of the composition.

Moreover, the inventors have observed that the composition according to the present invention has the advantage of providing users with a very advantageous sensory aspect. Thus, it has been observed that on application the skin appeared softer, more moisturized, firmer, more elastic and/or more plumped. It has also been observed that the composition according to the present invention helped to fortify and make the skin more resistant to help protect it against external aggressors like pollution and cold weather or against stress and fatigue. In other words, the composition reinforces the cutaneous barrier.

In addition, against all expectations and as demonstrated in the experimental section below, the inventors actually observed that these specific gelling polymers made it possible to ensure physicochemical stability of the compositions thus obtained.

The present invention also targets a process for the cosmetic treatment of keratin materials and especially the skin, and even more particularly the skin of the face, comprising at least the application, to said keratin materials, of a composition according to the invention.

The present invention further relates to a process for the cosmetic treatment as claimed in claim 13.

The present invention also relates to a process for the cosmetic treatment of an aged, especially wrinkled, skin, comprising the application, to said skin, of at least one composition according to the present invention, in an effective amount for toning down wrinkles.

The present invention also relates to a process for the cosmetic treatment of a dry skin, comprising the application, to said skin, of at least one composition according to the present invention, in an effective amount for improving the moisturization thereof.

The present invention also relates to a process for the cosmetic treatment of a skin affected by external aggressors like pollution and cold weather or by stress and fatigue, comprising the application, to said skin, of at least one composition according to the present invention, in an effective amount for improving the cutaneous barrier thereof.

Herein is described and not encompassed by the wording of the claims, the use of at least one acrylic acid polymer, of at least one copolymer of acrylic acid and of alkylacrylate or of at least one of the mixtures thereof, with a view to stabilizing a composition comprising hyaluronic acid or a salt thereof and spring and/or mineral water in a content greater than 20% by weight, or even than 25% by weight or else than 40% by weight relative to the total weight of said composition.

The composition according to the invention is advantageously cosmetic.

The term "cosmetic composition" is intended to mean a substance or a preparation intended to be brought into contact with the various superficial parts of the human body, especially the epidermis, the lips and the oral mucous membranes, with a view, exclusively or mainly, to cleansing them, making them more attractive, fragrancing them, modifying their appearance, protecting them, keeping them in good condition, or correcting body odors.

The composition according to the invention may comprise a "physiologically acceptable medium".

For the purposes of the present invention, the term "physiologically acceptable medium" is intended to mean a medium that is suitable for the topical administration of a composition, and that is compatible with all human keratin materials, such as the skin, the lips, the nails, the mucous membranes, the eyelashes, the eyebrows, the scalp and/or the hair, or any other area of bodily skin. According to the invention, a physiologically acceptable medium is preferentially a cosmetically acceptable medium, that is to say a medium which is free of any odor or unpleasant appearance, and which is entirely compatible with the topical administration route.

More particularly, the composition according to the invention is suitable for topical administration, that is to say for application to the surface of the keratin material under consideration, such as the skin.

According to the invention, the term "keratin materials" is intended to mean the skin, of the body, face and/or area around the eyes, the lips, the nails, the mucous membranes, the eyelashes, the eyebrows, bodily hair, the scalp and/or the hair, or any other area of bodily skin. More particularly, the keratin materials according to the invention are the scalp, the hair and/or the skin.

Preferably, the keratin material according to the invention is the skin.

The term "skin" is intended to mean all of the skin of the body, and preferably the skin of the face, neckline, neck, arms and forearms, or even more preferably still the skin of the face, especially of the forehead, nose, cheeks, chin and area around the eyes.

A composition according to the invention has an aqueous phase which advantageously constitutes its only fluid phase. As specified above, a composition according to the invention is advantageously a single-phase composition. In other words, it is different from an emulsion and is different from a composition comprising liposomes

The present invention is directed to a single-phase composition.

The present invention relates to a transparent composition, and more particularly to a transparent gel.

### SPRING AND/OR MINERAL WATER

For the purposes of the present invention, the term "mineralization" means the sum of the concentrations of anions and cations present in the spring or mineral water.

The present invention may use, without preference, a spring water or a mineral water or a mixture of the two. In general, a mineral water is fit for consumption, which is not always the case with a spring water. Each of these waters contains, *inter alia,* dissolved minerals and trace elements. These waters are known to be employed for specific treatment purposes according to the particular trace elements and minerals that they contain, such as the moisturization and desensitization of the skin or the soothing care of the skin.

The spring water or mineral water used according to the invention may in particular be chosen from the waters of the Vichy basin.

Among the waters of the Vichy basin, mention may be made of Lucas water, l'Hôpital water, and Grande Grille water.

According to a particular embodiment of the invention, the spring and/or mineral water may have a mineralization of greater than 400 mg/l, especially greater than 700 mg/l.

According to another particular embodiment of the present invention, the spring and/or mineral water have a mineralization of greater than 100 mg/l, 2000 mg/l, 3000 mg/l and even 4000mg/l.

The spring and/or mineral water used according to the invention may have a mineralization of at least 700 mg/l, and in particular have a total concentration of carbonates and bicarbonates of at least 150 mg/l and more preferentially of at least 360 mg/l and especially of sodium carbonate and bicarbonate of greater than 2 mg/l.

The concentration of silicon oxide in the water used in the composition according to the invention may preferably be at least 6 mg/l and more preferentially at least 9 mg/l.

According to a particular embodiment, the spring and/or mineral water is present in an amount greater than 25% by weight, preferably greater than 40% and better still ranging from 20% to 99%, or even ranging from 25% to 99%, or else ranging from 40% to 99 % by weight relative to the total weight of the composition. The spring or mineral water may be supplemented with distilled or demineralized water in the composition.

### HYALURONIC ACID

For the purposes of the present invention, hyaluronic acid also covers the salts thereof.

Within the context of the present invention, the term "hyaluronic acid or a salt thereof" especially covers the basic unit of hyaluronic acid, of formula:

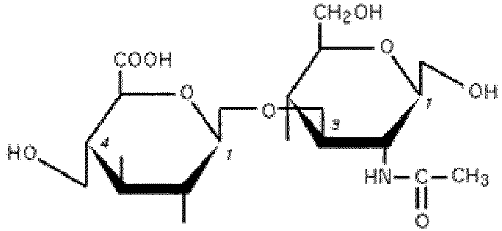

This is the smallest fraction of hyaluronic acid, comprising a disaccharide dimer, namely D-glucuronic acid and N-acetylglucosamine.

The term "hyaluronic acid or a salt thereof" also comprises, within the context of the present invention, the linear polymer comprising the polymeric unit described above, according to a sequence with alternating β(1,4) and β(1,3) glycosidic bonds, having a molecular weight (MW) which may vary between 380 and 13 000 000 daltons. This molecular weight mainly depends on the source from which the hyaluronic acid is obtained and/or the preparation methods.

The production of hyaluronic acid by bacteria has been known for more than 50 years, with the most commonly used microorganisms in this context being bacteria of the genus *Streptoccocus,* in particular those of the serological groups A (*Streptococcus pyogenes*) and C (*Streptococcus equi*) (Lancefield grouping).

The production of hyaluronic acid from cultures of recombinant microorganisms such as *Bacillus subtilis* (Brown and Pummill, 2008; Chien and Lee, 2007), *Escherichia Coli* (Yu and Stephanopoulos, 2008) and *Lactococcus lactis* (Chien and Lee, 2007) is also known.

After fermentation and various treatment steps (dilution, filtration, ethanol precipitation), crude hyaluronic acid is obtained.

A hyaluronic acid salt according to the invention is especially an alkali metal salt and may in particular be chosen from sodium hyaluronate and potassium hyaluronate.

It is preferably a sodium hyaluronate.

A hyaluronic acid or a salt thereof according to the present invention especially has a molecular weight of between 20 kDa and 5000 kDa, in particular between 500 kDa and 2200 kDa.

A hyaluronic acid or a salt thereof according to the invention may especially be provided:
- by SOLIANCE under the trade name CRISTALHYAL LO, RENOHYAL LO, GENHYAL
- by BLOOMAGE FREDA BIOPHARM under the trade name sodium hyaluronate (HA-T),
- by SOCHIBIO under the trade name Bio-sodium hyaluronate powder MMW or LMW, or
- by SHANDONG TOPSCIENCE BIOTECH under the trade name sodium hyaluronate.

The hyaluronic acid or a salt thereof is present in the composition in a dry matter content of between 0.05% and 1% by weight, in particular between 0.1 and 0.8% by weight, and even more particularly between 0.2 and 0.6% by weight relative to the total weight of the composition.

### ACRYLIC ACID POLYMERS OR COPOLYMER OF ACRYLIC ACID

### AND OF ALKYLACRYLATE

An acrylic polymer in accordance with the present invention is constituted of, a crosslinked homopolymer of acrylic acid.

The homopolymer may be crosslinked with a crosslinking agent, chosen especially from pentaerythrityl allyl ether, sucrose allyl ether and propylene allyl ether.

Such polymers have the INCI name: Carbomer.

Therefore, the present invention in particular concerns a composition comprising hyaluronic acid or a salt thereof, spring and/or mineral water in a content greater than 20% by weight relative to the total weight of the composition, and at least a homopolymer of crosslinked acrylic acid.

An acrylic acid polymer according to the present invention may especially be provided:
- by Lubrizol under the trade name CARBOPOL 981 (Carbomer),
- by ASHLAND under the trade name Ashland 980 MS (Carbomer),
- by Lubrizol under the trade name CARBOPOL 980 (carboxyvinyl polymer synthesized in an ethyl acetate/cyclohexane mixture),
- by Lubrizol under the trade name CARBOPOL ULTREZ 10 (crosslinked carboxyvinyl homopolymer synthesized in an ethyl acetate/cyclohexane mixture),
- by 3V under the trade name SYNTHALEN K (carboxyvinyl polymer synthesized in methylene chloride),
- by 3V under the trade name SYNTHALEN L (Carbomer), or else
- by 3V under the trade name SYNTHALEN M (Carbomer).

A copolymer of acrylic acid and of alkylacrylate is defined as being a copolymer of (i) monomer of the following formula (I): 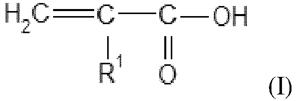
wherein R¹ denotes H or CH₃ or C₂H₅, and of (ii) monomer of the following formula (II) (monomer of (C₁₀-C₃₀)alkyl ester of unsaturated carboxylic acid type):

   H₂C=CR¹-COOR³ (II)
wherein R¹, not necessarily the same as for formula (I),denotes H or CH₃ or C₂H₅, which corresponds to acrylic acid, methacrylic acid or ethacrylic acid units, and preferably H (acrylate units) or CH₃ (methacrylate units), and R³ denotes a C₁₀-C₃₀ and preferably C₁₂-C₂₂ alkyl group.

In this polymer, the monomer (I) constitutes the hydrophilic unit and the monomer (II) constitutes the hydrophobic unit.

(C₁₀-C₃₀)Alkyl esters of unsaturated carboxylic acids comprise, for example, lauryl (meth)acrylate, stearyl (meth)acrylate, decyl (meth)acrylate, isodecyl (meth)acrylate and dodecyl (meth)acrylate.

Anionic polymers of this type are described and prepared, for example, according to patents US 3 915 921 and US 4 509 949.

Among this type of anionic polymers, use will more particularly be made of polymers formed from a mixture of monomers comprising:
(i) acrylic acid,
(ii) an ester of formula (II) described above in which R¹ denotes H or CH₃ and R³ denotes an alkyl group having from 12 to 22 carbon atoms,
(iii) and optionally a crosslinking agent, which is a well-known copolymerizable polyethylenic unsaturated monomer, such as diallyl phthalate, allyl (meth)acrylate, divinylbenzene, (poly)ethylene glycol dimethacrylate or methylenebisacrylamide.

Among this type of anionic polymers, use will more particularly be made of those constituted of from 95 to 60% by weight of acrylic acid (hydrophilic unit), 4 to 40% by weight of C₁₀-C₃₀ alkyl acrylate (hydrophobic unit) and 0 to 6% by weight of crosslinking polymerizable monomer, or alternatively those constituted of from 98 to 96% by weight of acrylic acid (hydrophilic unit), 1 to 4% by weight of C₁₀-C₃₀ alkyl acrylate (hydrophobic unit) and 0.1 to 0.6% by weight of crosslinking polymerizable monomer such as those described above.

Said above polymers may especially be provided:
- by Lubrizol under the trade name PEMULEN TR-1 POLYMER (acrylic acid/stearyl methacrylate copolymer polymerized in an ethyl acetate/cyclohexane mixture),
- by Lubrizol under the trade name PEMULEN TR-2 (crosslinked acrylic acid/alkyl acrylate polymer),
- by Lubrizol under the trade name CARBOPOL 1382 (acrylic acid/stearyl methacrylate copolymer polymerized in an ethyl acetate/cyclohexane mixture),
- by Lubrizol under the trade name CARBOPOL ETD 2020 (crosslinked acrylates/C10-C30 alkyl acrylates polymer),
- by Lubrizol under the trade name CARBOPOL ULTREZ 21 (crosslinked acrylic-acrylates/C10-C30 alkyl acrylate polymer),
- by Lubrizol under the trade name CARBOPOL ULTREZ 20 (crosslinked acrylates/C10-C30 alkyl acrylate polymer),
- by Lubrizol under the trade name CARBOPOL ULTREZ 10 (crosslinked carboxyvinyl homopolymer synthesized in an ethyl acetate/cyclohexane mixture).

According to the present invention, preference is most particularly given, as acrylic acid polymers or copolymers of acrylic acid and of alkylacrylate, to the products sold by Lubrizol under the trade names CARBOPOL 980, 981 and by 3V under the trade name SYNTHALEN K.

The acrylic acid polymer, the copolymer of acrylic acid and of alkylacrylate or a mixture thereof is present in the composition in a dry matter content of between 0.01% and 5%, in particular between 0.05 and 4% by weight, and even more particularly between 0.1 and 2% by weight relative to the total weight of the composition.

According to a particular embodiment of the invention, the composition comprises water of the Vichy basin in a content greater than 20% by weight relative to the total weight of the composition, sodium hyaluronate and an acrylic polymer.

### COMPOSITION AND ADDITIVES

The composition of the present invention is in the form of a gel and even more in the form of a transparent gel.

According to a particular embodiment, the composition according to the present invention is free of base, and more precisely is free of an additional base.

According to a particular embodiment, the composition according to the present invention may also contain an organic acid.

Indeed, according to a particular embodiment of the invention, the composition has a pH which may be between 4.5 and 7.

Among these acids, mention may especially be made of citric acid, salicylic acid and derivatives thereof (LHA), lactic acid and mixtures thereof.

According to an even more preferred embodiment, the composition according to the present invention also comprises citric acid.

The additional active agents for cosmetic treatment of keratin materials, such as the skin, of use in the compositions of the invention may especially be chosen from desquamating agents, antimicrobial agents, soothing agents, antioxidants, astringents, anti-aging agents, firming agents, anti wrinkle agents, moisturizers, and mixtures thereof.

The composition of the invention more particularly constitutes a moisturizing and/or anti-aging, in particular antiwrinkle, facial care composition. It may nonetheless, as a variant, constitute a body care composition.

The composition of the invention may constitute a fortifying and hydrating daily skin booster.

The composition may also help to soothe and strengthen the skin against external aggressors and namely contribute to fortify and make the skin more resistant to help protect it against external aggressors like pollution and cold weather.

The composition according to the present invention may be applied daily on the skin.

After a few weeks of daily use, it may be observed that the skin is more hydrated and appears plumped and toned. As a whole, the skin appears with an improved healthy-looking glow.

The additional, advantageously hydrophilic active agent(s) may be present in the composition according to the invention in a content ranging from 0.001% to 20% by weight, preferably ranging from 0.01% to 5% by weight and preferentially ranging from 0.05% to 2% by weight relative to the total weight of the composition.

According to a variant, the composition according to the invention may be a care composition, especially a skincare product such as a care gel (day care, night care, anti wrinkle care) or else a care mask.

### Secondary ingredients

A composition according to the invention may also contain one or more colorants, of water-soluble pigment or dye type.

The composition according to the invention may also comprise adjuvants that are common in the cosmetics and dermatology fields, such as fragrances and preservatives. The amounts of these various adjuvants are those conventionally used in the fields under consideration, and for example from 0.01% to 20% of the total weight of the composition.

Needless to say, those skilled in the art will take care to select the optional compound(s) to be added to the compositions according to the invention and also the concentration thereof, such that the advantageous properties intrinsically associated with the compositions in accordance with the invention are not, or are not substantially, adversely affected by the envisioned addition.

Throughout the description, including the claims, the term "*comprising a*" should be understood as being synonymous with "*comprising at least one*", unless otherwise specified.

The terms "*between... and...*" and "*ranging from... to...*" should be understood as being inclusive of the limits, unless otherwise specified.

In the description and the examples, the percentages are percentages by weight, unless otherwise indicated. The percentages are thus given by weight relative to the total weight of the composition. The ingredients are mixed in the order and under the conditions that are easily determined by those skilled in the art.

### EXAMPLES

The viscosity was measured with a Lamy Rheomat RM100. The measurements are given in UD and measured with spindles 3 (M3) or 2 (M2).

### Example 1: transparent aqueous gel

Procedure: The aqueous gel is prepared under cold conditions with stirring. The addition of the phase E (citric acid) into the phase A (aqueous phase) precedes the addition of the glycols. The Carbomer is added last. The citric acid may be added either into the aqueous phase or at the end of formulation. The sodium hyaluronate may be introduced at the same time as the carbomer.

| **Phase** | **ingredients** | **Concentration in % by weight** |
|---|---|---|
| A | Water of Vichy basin | **88.65** |
| A | Preservatives | **1** |
| A | Glycols | **5** |
| A | Moisturizers | **4** |
| B | Sodium hyaluronate ¹ | **0.4** |
| C | CARBOMER ² | **0.6** |
| E | Citric acid | **0.35** |

| | | |
|---|---|---|
| ¹ sold by SOLIANCE under the trade name crystalhyal LO ² sold by Lubrizol under the trade name CARBOPOL 980 | | |

### Physicochemical characterization

The pH was measured at 5.3.

The viscosity was also measured:
Viscosity Pa.s (Poise) T0-T10 min 24 UD (M3 = 0.78 Pa.s (7.8 poise) 23 UD (M3) = 0.73 Pa.s (7.3 poise)

### Example 2: transparent aqueous gel

The present aqueous gel may be prepared according to the protocol described in example 1.

| **Phase** | **ingredients** | **Concentration in % by weight** |
|---|---|---|
| A | Water of Vichy basin | **97.2** |
| A | Preservatives | **0.7** |
| B | Sodium hyaluronate ¹ | **0.1** |
| C | CARBOMER ² | **2** |

| | | |
|---|---|---|
| ¹ sold by BLOOMAGE FREDA BIOPHARM under the trade name sodium hyaluronate ² sold by 3V under the trade name SYNTHALEN K | | |

### Physicochemical characterization

The pH was measured at 4.8.

The viscosity was also measured:
Viscosity T0/T10 min 29 UD (M4) = 6.9 Pa.s (69 poises) /28 UD (M4) = 6.6 Pa.s (66 poises)

### Example 3: transparent aqueous gel

The present aqueous gel may be prepared according to the protocol described in example 1.

| **Phase** | **ingredients** | **Concentration in % by weight** |
|---|---|---|
| A | Water of Vichy basin | **98.2** |
| A | Preservative | **0.7** |
| A | CITRIC ACID | **0.1** |
| B | Sodium hyaluronate ¹ | **0.5** |
| C | CARBOMER ² | **0.5** |

| | | |
|---|---|---|
| ¹ sold by SOLIANCE under the trade name crystalhyal LO ² sold by Lubrizol under the trade name CARBOPOL 981 | | |

### Physicochemical characterization

The pH was measured at 6.8.

The viscosity was also measured:
Viscosity T0/T10 min 79 UD (M2) = 0.57 Pa.s (570 cps) cps/ 78 UD (M2) = 0.562 Pa.s (562 cps)

### Conclusion

The gels obtained in examples 1 to 3 above all have satisfactory stability. No phase separation was thus observed, nor the appearance of crystals, which may be observed when mixing hyaluronic acid with a spring and/or mineral water when the content thereof exceeds especially 10% and even more so 15% by weight relative to the total weight of the composition employing same.

Applied to the skin of the face, including the area around the lips and the eyes, such compositions make it possible to provide moisture and firmness.

## Claims

1. A composition comprising hyaluronic acid or a salt thereof, spring and/or mineral water in a content greater than 20% by weight relative to the total weight of the composition, and at least one acrylic acid polymer, a copolymer of acrylic acid and of alkylacrylate, or one of the mixtures thereof, wherein the composition is in a single phase, and wherein it is provided in the form of a gel, **characterized in that** the gel is transparent, wherein the hyaluronic acid or a salt thereof is present in a dry matter content of between 0.05% and 1 % by weight relative to the total weight of the composition,
wherein the acrylic acid polymer is a homopolymer of crosslinked acrylic acid and the copolymer of acrylic acid and of alkylacrylate is chosen from the copolymers of (i) monomer of the following formula (I): 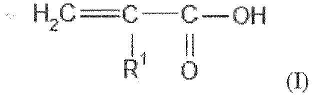 wherein R¹ denotes H or CH₃ or C₂H₅, and of (ii) monomer of the following formula (II):
H₂C=CR¹-COOR³ (II)
wherein R¹ is same as or different from that in formula (I), denotes H or CH₃ or C₂H₅, R³ denotes a C₁₀-Ca₃₀, alkyl group,
wherein the acrylic acid polymer, the copolymer of acrylic acid and of alkylacrylate or a mixture thereof is present in the composition in a dry matter content of between 0.01% and 5% by weight, relative to the total weight of the composition.

2. The composition as claimed in claim 1, wherein the hyaluronic acid or a salt thereof is present in a dry matter content of between 0.1 and 0.8% by weight, and even more particularly between 0.2 and 0.5% by weight relative to the total weight of the composition.

3. The composition as claimed in claim 1 or 2, wherein the hyaluronic acid salt is chosen from an alkali metal salt, for example sodium hyaluronate and potassium hyaluronate, and is preferably sodium hyaluronate.

4. The composition as claimed in anyone of the preceding claims, wherein the copolymer of acrylic acid and of alkylacrylate is formed from a mixture of monomers comprising:
(i) acrylic acid,
(ii) an ester of formula (II) as defined in claim 4 and wherein R¹ denotes H or CH₃ and R³ denotes an alkyl group having from 12 to 22 carbon atoms,
(iii) and optionally a crosslinking agent, which is a copolymerizable polyethylenic unsaturated monomer chosen from diallyl phthalate, allyl (meth)acrylate, divinylbenzene, (poly)ethylene glycol dimethacrylate or methylenebisacrylamide.

5. The composition as claimed in any one of the preceding claims, wherein it comprises hyaluronic acid or a salt thereof, spring and/or mineral water in a content greater than 20% by weight relative to the total weight of the composition, and at least a homopolymer of crosslinked acrylic acid.

6. The composition as claimed in any one of the preceding claims, wherein the acrylic acid polymer, the copolymer of acrylic acid and of alkylacrylate or a mixture thereof is present in the composition in a dry matter content of between 0.05 and 4% by weight, and in particular between 0.1 and 2% by weight relative to the total weight of the composition.

7. The composition as claimed in any one of the preceding claims, wherein the spring and/or mineral water has a mineralization of greater than 400 mg/l, especially greater than 700 mg/l, greater than 1000 mg/l, 2000 mg/l, 3000 mg/l or 4000 mg/l.

8. The composition as claimed in any one of the preceding claims, wherein the spring and/or mineral water has a total concentration of carbonate and bicarbonate ions of at least 150 mg/l and preferentially of at least 360 mg/l and especially of sodium carbonate and bicarbonate of greater than 2 mg/l.

9. The composition as claimed in any one of the preceding claims, wherein the spring and/or mineral water has a silicon oxide concentration of at least 6 mg/l and especially of at least 9 mg/l.

10. The composition as claimed in any one of the preceding claims, wherein the spring and/or mineral water is chosen from a water spring of the Vichy basin, in particular chosen from Grande Grille water, l'Hopital water, and Lucas water.

11. The composition as claimed in any one of the preceding claims, **characterized in that** the spring and/or mineral water is present in an amount greater than 25% by weight, preferably greater than 40% and better still ranging from 20% to 99%, or even ranging from 25 to 99%, or else ranging from 40% to 99% by weight relative to the total weight of the composition.

12. The composition as claimed in any one of the preceding claims, **characterized in that** it also contains an organic acid, especially chosen from citric acid, salicylic acid and derivatives thereof, lactic acid, and mixtures thereof, and more particularly citric acid.

13. A process for the cosmetic treatment of keratin materials and especially the skin, and even more particularly the skin of the face, comprising at least the application, to said keratin materials, of a composition as defined in any one of the preceding claims, in particular for the cosmetic treatment of an aged, especially wrinkled, skin, comprising the application to said skin of at least said composition in an effective amount for toning down wrinkles or in particular for the cosmetic treatment of a dry skin, comprising the application, to said skin, of at least said composition, in an effective amount for improving the moisturization thereof or even in particular for the cosmetic treatment of a skin affected by external aggressors like pollution and cold weather or by stress and fatigue, comprising the application, to said skin, of at least said composition, in an effective amount for improving the cutaneous barrier thereof.

## Patentansprüche

1. Zusammensetzung, umfassend Hyaluronsäure oder ein Salz davon, Quell- und/oder Mineralwasser mit einem Gehalt von mehr als 20 Gew.-%, bezogen auf das Gesamtgewicht der Zusammensetzung, und mindestens ein Acrylsäurepolymer, ein Copolymer aus Acrylsäure und aus Alkylacrylat, oder eine der Mischungen davon, wobei die Zusammensetzung in einer einzigen Phase vorliegt, und wobei sie in Form eines Gels bereitgestellt ist, **dadurch gekennzeichnet, dass** das Gel transparent ist, wobei die Hyaluronsäure oder ein Salz davon mit einem Trockenmassegehalt von zwischen 0,05 Gew.-% und 1 Gew.-% bezogen auf das Gesamtgewicht der Zusammensetzung vorliegt,
wobei das Acrylsäurepolymer ein Homopolymer aus vernetzter Acrylsäure ist und das Copolymer aus Acrylsäure und Alkylacrylat ausgewählt ist aus den Copolymeren von (i) Monomer der folgenden Formel (I): 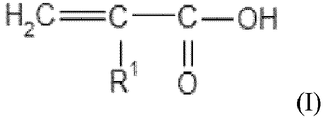
wobei R¹ H oder CH₃ oder C₂H₅ bezeichnet, und von (ii) Monomer der folgenden Formel (II):
H₂C=CR¹-COOR³ (II)
wobei R¹ gleich oder verschieden von dem in Formel (I) ist, H oder CH₃ oder C₂H₅ bezeichnet, R³ eine C₁₀-C₃₀ Alkylgruppe bezeichnet,
wobei das Acrylsäurepolymer, das Copolymer aus Acrylsäure und aus Alkylacrylat oder eine Mischung davon in der Zusammensetzung mit einem Trockenmassegehalt von zwischen 0,01 und 5 Gew.-% bezogen auf das Gesamtgewicht der Zusammensetzung vorliegt.

2. Zusammensetzung nach Anspruch 1, wobei die Hyaluronsäure oder ein Salz davon mit einem Trockenmassegehalt zwischen 0,1 und 0,8 Gew.-% vorliegt, und noch bevorzugter zwischen 0,2 und 0,5 Gew.-%, bezogen auf das Gesamtgewicht der Zusammensetzung.

3. Zusammensetzung nach Anspruch 1 oder 2, wobei das Hyaluronsäuresalz ausgewählt ist aus einem Alkalimetallsalz, z. B. Natriumhyaluronat und Kaliumhyaluronat, und bevorzugt Natriumhyaluronat ist.

4. Zusammensetzung nach einem der vorangehenden Ansprüche, wobei das Copolymer aus Acrylsäure und Alkylacrylat aus einer Mischung von Monomeren gebildet ist, umfassend:
(i) Acrylsäure,
(ii) einem Ester der Formel (II), wie in Anspruch 4 definiert, und wobei R¹ H oder CH₃ bezeichnet und R³ eine Alkylgruppe mit 12 bis 22 Kohlenstoffatomen bezeichnet,
(iii) und optional ein Vernetzungsmittel, welches ein copolymerisierbares polyethylenisch ungesättigtes Monomer, ausgewählt aus Diallylphthalat, Allyl(meth)acrylat, Divinylbenzol, (Poly)ethylenglykoldimethacrylat oder Methylenbisacrylamid, ist.

5. Zusammensetzung nach einem der vorhergehenden Ansprüche, wobei diese Hyaluronsäure oder ein Salz davon, Quell- und/oder Mineralwasser mit einem Gehalt von mehr als 20 Gew.-% bezogen auf das Gesamtgewicht der Zusammensetzung und mindestens ein Homopolymer der vernetzten Acrylsäure umfasst.

6. Zusammensetzung nach einem der vorhergehenden Ansprüche, wobei das Acrylsäurepolymer, das Copolymer aus Acrylsäure und Alkylacrylat oder eine Mischung davon in der Zusammensetzung mit einem Trockenmassegehalt zwischen 0,05 und 4 Gew.-% vorliegen, und insbesondere zwischen 0,1 und 2 Gew.-%, bezogen auf das Gesamtgewicht der Zusammensetzung.

7. Zusammensetzung nach einem der vorhergehenden Ansprüche, wobei das Quell- und/oder Mineralwasser einen Mineralisierungsgrad von mehr als 400 mg/l, insbesondere mehr als 700 mg/l, mehr als 1000 mg/l, 2000 mg/l, 3000 mg/l oder 4000 mg/l aufweist.

8. Zusammensetzung nach einem der vorhergehenden Ansprüche, wobei das Quell- und/oder Mineralwasser eine Gesamtkonzentration an Carbonat- und Bicarbonationen von mindestens 150 mg/l und vorzugsweise mindestens 360 mg/l, insbesondere von Natriumcarbonat und -bicarbonat von mehr als 2 mg/l, aufweist.

9. Zusammensetzung nach einem der vorhergehenden Ansprüche, wobei das Quell- und/oder Mineralwasser eine Siliziumoxid-Konzentration von mindestens 6 mg/l und insbesondere von mindestens 9 mg/1 aufweist.

10. Zusammensetzung nach einem der vorhergehenden Ansprüche, wobei das Quell- und/oder Mineralwasser aus einer Wasserquelle des Vichy-Beckens ausgewählt ist, insbesondere aus Grande Grille-Wasser, l'Hôpital-Wasser und Lucas-Wasser ausgewählt ist.

11. Zusammensetzung nach einem der vorangehenden Ansprüche, **dadurch gekennzeichnet, dass** das Quell- und/oder Mineralwasser in einer Menge, die größer ist als 25 Gew.-%, vorzugsweise größer als 40 % und besser noch zwischen 20 % und 99 %, oder sogar zwischen 25 und 99 % oder zwischen 40 % und 99 % bezogen auf das Gesamtgewicht der Zusammensetzung vorliegt.

12. Zusammensetzung nach einem der vorangehenden Ansprüche, **dadurch gekennzeichnet, dass** diese auch eine organische Säure enthält, insbesondere ausgewählt aus Zitronensäure, Salicylsäure und Derivaten davon, Milchsäure und Mischungen davon, und insbesondere Zitronensäure.

13. Verfahren für die kosmetischen Behandlung von Keratinmaterialien und insbesondere der Haut, insbesondere der Gesichtshaut, das mindestens die Auftragung einer Zusammensetzung nach einem der vorhergehenden Ansprüche, auf diese Keratinmaterialien, umfasst, insbesondere für die kosmetische Behandlung einer gealterten, insbesondere faltigen Haut, umfassend die Auftragung mindestens dieser Zusammensetzung in einer wirksamen Menge auf diese Haut, um Falten zu reduzieren, oder insbesondere für die kosmetischen Behandlung einer trockenen Haut, umfassend die Auftragung mindestens dieser Zusammensetzung auf diese Haut in einer wirksamen Menge, um die Befeuchtung davon zu verbessern, oder auch insbesondere für die kosmetische Behandlung einer Haut, die durch externe Aggressoren wie Umweltverschmutzung und kaltes Wetter oder durch Stress und Müdigkeit beeinträchtigt ist, umfassend die Auftragung mindestens der genannten Zusammensetzung in einer wirksamen Menge auf diese Haut zur Verbesserung der Hautbarriere davon.

## Revendications

1. Composition comprenant de l'acide hyaluronique ou l'un de ses sels, de l'eau de source et/ou minérale dans une teneur supérieure à 20% en poids par rapport au poids total de la composition ainsi qu'au moins un polymère d'acide acrylique, un copolymère d'acide acrylique et d'acrylate d'alkyle ou un de leurs mélanges, laquelle composition est monophasique et se présente sous la forme d'un gel, **caractérisée en ce que** le gel est transparent,
dans laquelle l'acide hyaluronique ou l'un de ses sels est présent en une quantité de matière sèche comprise entre 0,05 % et 1 % en poids par rapport au poids total de la composition,
dans laquelle le polymère d'acide acrylique est un homopolymère d'acide acrylique réticulé et le copolymère d'acide acrylique et d'acrylate d'alkyle est choisi parmi les copolymères de (i) monomère de formule (I) suivante : 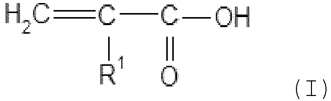
dans laquelle R¹ désigne H ou CH₃ ou C₂H₅, et de (ii) monomère de formule (II) suivante :
H₂C=CR¹-COOR³ (II)
dans laquelle R¹ est identique à ou différent de celui dans la formule (I) et désigne H ou CH₃ ou C₂H₅, R³ désigne un groupe alkyle en C₁₀-C₃₀,
dans laquelle le polymère d'acide acrylique, le copolymère d'acide acrylique et d'acrylate d'alkyle ou un de leurs mélanges est présent dans la composition dans une teneur en matière sèche comprise entre 0,01 % et 5 % en poids par rapport au poids total de la composition.

2. Composition selon la revendication 1, dans laquelle l'acide hyaluronique ou l'un de ses sels est présent dans une teneur en matière sèche comprise entre 0,1 et 0,8 % en poids, et encore plus particulièrement entre 0,2 et 0,5% en poids par rapport au poids total de la composition.

3. Composition selon la revendication 1 ou 2, dans laquelle le sel d'acide hyaluronique est choisi parmi un sel de métal alcalin, par exemple parmi le hyaluronate de sodium et le hyaluronate de potassium, et de préférence est le hyaluronate de sodium.

4. Composition selon l'une quelconque des revendications précédentes, dans laquelle le copolymère d'acide acrylique et d'acrylate d'alkyle est formé à partir d'un mélange de monomères comprenant :
(i) de l'acide acrylique,
(ii) un ester de formule (II) tel que défini en revendication 4 et dans lequel R¹ désigne H ou CH₃, et R³ désigne un groupe alkyle ayant de 12 à 22 atomes de carbone,
(iii) et éventuellement un agent réticulant, qui est un monomère insaturé polyéthylénique copolymérisable choisi parmi le phtalate de diallyle, le (méth)acrylate d'allyle, le divinylbenzène, le diméthacrylate de (poly)éthylèneglycol, et le méthylène-bisacrylamide.

5. Composition selon l'une quelconque des revendications précédentes, qui comprend de l'acide hyaluronique ou l'un de ses sels, de l'eau de source et/ou minérale dans une teneur supérieure à 20% en poids par rapport au poids total de la composition ainsi qu'au moins un homopolymère d'acide acrylique réticulé.

6. Composition selon l'une quelconque des revendications précédentes, dans laquelle le polymère d'acide acrylique, le copolymère d'acide acrylique et d'acrylate d'alkyle ou un de leurs mélanges est présent dans la composition dans une teneur en matière sèche comprise entre 0,05 et 4 % en poids, et en particulier entre 0,1 et 2 % en poids par rapport au poids total de la composition.

7. Composition selon l'une quelconque des revendications précédentes, dans laquelle l'eau de source et/ou minérale possède une minéralisation supérieure à 400 mg/l, notamment supérieure à 700 mg/l, supérieure à 1000 mg/l, 2000 mg/l, 3000 mg/l ou 4000 mg/l.

8. Composition selon l'une quelconque des revendications précédentes, dans laquelle l'eau de source et/ou minérale possède une concentration totale en ions carbonates et bicarbonates d'au moins 150 mg/l, et préférentiellement d'au moins 360 mg/l, et notamment en carbonate et bicarbonate de sodium supérieure à 2 mg/l.

9. Composition selon l'une quelconque des revendications précédentes, dans laquelle l'eau de source et/ou minérale a une concentration en oxyde de silicium d'au moins 6 mg/l, et notamment d'au moins 9 mg/l.

10. Composition selon l'une quelconque des revendications précédentes, dans laquelle l'eau de source et/ou minérale est choisie parmi une source d'eau du bassin de Vichy, en particulier choisie parmi l'eau de Grande Grille, l'eau d'Hôpital, et l'eau de Lucas.

11. Composition selon l'une quelconque des revendications précédentes, **caractérisée en ce que** l'eau de source et/ou minérale est présente dans une teneur supérieure à 25 % en poids, de préférence supérieure à 40 % et mieux allant de 20 à 99 %, voire allant de 25 à 99 %, ou encore allant de 40 % à 99 % en poids par rapport au poids total de la composition.

12. Composition selon l'une quelconque des revendications précédentes, **caractérisée en ce qu'**elle contient en outre un acide organique, notamment choisi parmi l'acide citrique, l'acide salicylique et ses dérivés, l'acide lactique et leurs mélanges, et plus particulièrement l'acide citrique.

13. Procédé de traitement cosmétique des matières kératiniques et notamment de la peau, et encore plus particulièrement de la peau du visage, comprenant au moins l'application sur lesdites matières kératiniques d'une composition telle que définie dans l'une quelconque des revendications précédentes, en particulier de traitement cosmétique d'une peau vieillie, notamment ridée, comprenant l'application sur ladite peau d'au moins ladite composition en une quantité efficace pour estomper les rides, ou en particulier de traitement cosmétique d'une peau sèche, comprenant l'application sur ladite peau d'eau moins ladite composition, en une quantité efficace pour améliorer son hydratation, ou même en particulier de traitement cosmétique d'une peau affectée par des agresseurs externes tels que la pollution et le climat froid ou par le stress et la fatigue, comprenant l'application sur ladite peau d'eau moins ladite composition, en une quantité efficace pour améliorer sa barrière cutanée.
